# EUROPEAN PATENT SPECIFICATION

(11) **EP 1 136 088 B1**
(45) Date of publication and mention of the grant of the patent: **10.08.2005**
(21) Application number: 01201382.7
(22) Date of filing: 28.06.1995
(51) Int. Cl.: A61M 5/14, A61M 5/168, F16M 13/00

(54) **Medical pressure transducer with sliding components**
Medizinischer Druckwandler mit Schiebekomponenten
Transducteur de pression médical avec des composants coulissants

(43) Date of publication of application: 26.09.2001
(62) Divisional of application: 95925359.2
(73) Proprietor: MEDEX, INC., Hilliard Ohio 43026 (US)
(72) Inventor: Fowler, James H, Plain City, Ohio 43064 (US); Patzer, Charles R, Ashville, Ohio 43103 (US); Nicholson, Warren B, Saginaw, MI 48609 (US); Thompson, Wendell, Dublin, Ohio 43016 (US); Brunner, Glenn D, Dublin, Ohio 43016 (US); Adams, Theodore R, Amlin, Ohio 43002 (US); Shah, Nilesh M, Springdale, Ohio 45246 (US)
(74) Representative: Findlay, Alice Rosemary

(56) References cited:
- WO-A-95/01195
- US-A- 4 920 972
- US-A- 4 970 900
- US-A- 5 257 917
- US-A- 5 752 918

## Description

This invention relates to medical pressure transducers, and more particularly, to such transducers in which a disposable fluid path component such as a dome is selectively attachable to a reusable sensor component with respective fluid pressure communicating diaphragms of the components in confronting, pressure communicating relationship.

### Background of the Invention

In hospital environments, for example, many procedures involve monitoring bodily fluid pressures such as blood pressure. Typically, such pressure is monitored with a medical pressure transducer outside the patient's body and coupled hydraulically to the patient's circulatory system, by way of example, via a catheter introduced into the body. The catheter is coupled via a tube to a fluid path inside the transducer and the tube is filled with saline to hydraulically communicate pressure within the patient's body to the transducer.

The transducer includes a sensor in pressure communication with the fluid path by which to convert the pressure therein to electrical signals corresponding to the pressure. The electrical signals are coupled via a cable to a monitor which provides a visual display of the pressure.

US Patent No. 4970900 discloses a module system for mounting medical devices on an IV pole at a patient's bedside. Each module may include a transducer. The modules are mounted to a receptacle which in turn is connected to the IV pole.

One particularly successful form of such a transducer is provided by a two-component system in which one component with the expensive sensor is reusable, and the other component with the patient-contacting fluid path is disposable. Each component is provided with a diaphragm closing off access to the sensor or the fluid path, respectively. To measure pressure in the fluid path, the disposable component is screwed onto the reusable component with the diaphragms in confronting, pressure communicating relationship to thereby communicate pressure from the fluid path to the sensor. After use, the disposable component is unscrewed from the reusable part and discarded, and replaced with a new, sterile unit.

U.S. Patent 4920972 shows an example of a two-component transducer in which the disposable fluid path component, referred to as a fluid dome, is rotatably coupled to the reusable sensor portion. The components are secured together by threaded interaction to bring the diaphragms into confronting, pressure communication relationship. Pressure from the fluid path in the dome is thus communicated through the dome diaphragm and reusable diaphragm and through a cured gel to the sensor of the reusable component. The screwed-together components may then be mounted to a supporting plate to be attached to a pole as is conventional.

Another example of a two-component transducer is shown in International Patent Application WO95/01195.

As shown in that publication and particularly with respect to Fig. 11 thereof, the dome is provided with wings that extend outwardly to opposite sides of the dome diaphragm to be slidably received into channels defined on a reusable component containing the sensor and reusable diaphragm. The channels are also to either side of the reusable diaphragm so that the dome diaphragm may be easily and quickly slid into confronting relationship with the sensor or reusable diaphragm. In particular, the reusable component may be formed as the supporting plate thus eliminating that extra part and the extra manipulation thereof by the operator. However, in the sliding wing and channel arrangement, improvements are desired to facilitate operator use of the transducer and to enhance the confronting, pressure transmitting relationship of the diaphragms.

A medical pressure transducer, in accordance with one aspect of the invention, comprises a reusable component for use with a disposable dome having a fluid path adapted to be coupled to a patient, a diaphragm coupled to said fluid path, and at least one mounting wing extending to one side of said dome diaphragm, the reusable component comprising a support, a pressure sensor permanently associated with the support, a reusable diaphragm in pressure communication with the sensor and at least one channel disposed to one side of the reusable diaphragm for slidably receiving said dome wing such that said dome diaphragm is slid by the reusable diaphragm and slidably received into confronting relationship with the reusable diaphragm, characterised by one of a depression and a projection associated with the support and being relatively positioned to receive a projection or be received in a depression, respectively, associated with said dome as said dome wing is slidably received into the channel to lock said disposable diaphragm into confronting relationship with the reusable diaphragm.

A medical pressure transducer, in accordance with another aspect of the invention, comprises a disposable dome for use with a reusable component having a support, a pressure sensor permanently associated with said support, a reusable diaphragm in pressure communication with said sensor and at least one channel disposed to one side of said reusable diaphragm, the disposable dome comprising a fluid path adapted to be coupled to a patient, a diaphragm coupled to the fluid path, and at least one mounting wing extending to one side of the dome diaphragm and slidably receivable in said reusable component channel such that the dome diaphragm slid by the reusable diaphragm and is slidably received into confronting relationship with said reusable diaphragm, characterised by one of a projection and a depression associated with the dome and being relatively positioned to be received in a depression or receive a projection, respectively, associated with said support as the wing is slidably received into said channel to lock the disposable diaphragm into confronting relationship with said reusable diaphragm.

The depression may be a tab-receiving slot formed in the reusable component and the projection locking tab associated with the disposable dome (or vice versa), the tab and slot engaging together to lock the dome to the reusable component as the dome wings are slidingly received into the channels. In the preferred embodiment, the slot is formed into the outer front wall and the locking tab is elevated above the associated wing on the dome so as to fit into the slot when the diaphragms are in confronting relationship. As a consequence, the dome and reusable components are reliably locked together as if they had been screwed together but without the same dexterity of manipulation required.

The medical pressure transducer preferably comprises both a disposable dome and a reusable component as above-described.

Camming structure may be associated with at least one of the channel and the dome wing by which to drive the disposable dome diaphragm as the dome wing is slidingly received into the channel.

Provision of the camming structure allows the two diaphragms to initially be slightly spaced apart, or loosely contacting, through at least part of the travel of the dome into the reusable component. In this way, the diaphragms are not significantly damaged or chafed as they slide by one another. Yet, the camming structure brings the diaphragms into abutting relationship, at least at the end of the travel of the components, such that proper pressure communication is established therebetween. To this end, and in a preferred embodiment, the camming structure is provided by one or more camming ramps at the terminal end of the sliding travel of the components. One such ramp may be formed as a step at the bottom end of the channel that is last contacted by the dome wing as it reaches the end of its travel. In the preferred embodiment, the channel is defined behind an outer front wall. In this regard, another such ramp may be formed on the mounting wing at the top end that is last contacted by the outer front wall as the wing reaches the end of its travel.

The medical pressure transducer also preferably comprises a disposable dome and a reusable component as above-described with the channel defined behind the outer front wall, and which are further characterised by a second wing associated with the dome and spaced above the mounting wing to define a wall-receiving space for the channel-defining outer front wall. In the preferred embodiment, the second wing overlies and generally conceals the outer wall. As a consequence, the assembled transducer does not have the appearance of being in two parts, but instead appears as a solid unit. The overlying relationship between the outer wall and second wing may also provide protection against contaminants entering and fouling the reusable component channel.

In the preferred embodiment, there are two such channels and two such mounting wings to fit slidingly into the channels with the channels and mounting wings situated to opposite sides of their respective diaphragms. In the preferred embodiment, there are also provided two second wings, the lateral side edges of which are indented and textured (such as by grooving, serrating or knurling) to provide finger-gripping portions by which the operator may manipulate the dome to slide it into and out of the channels. One of the indentations may be simulated with a similarly shaped and textured paddle to carry the locking tab. As a consequence, gripping the second wings serves also to compress the paddle thereby disengaging the tab from the slot and allowing sliding removal of the dome from the reusable component.

By virtue of the foregoing, there is thus provided a medical pressure transducer with a disposable fluid dome slidably removable to a reusable sensor component with an enhanced pressure transmitting relationship between the diaphragms and with improvements to facilitate operator use of the transducer.

These and other objects and advantages of the invention shall be made apparent from the accompanying drawings and descriptions thereof.

### Brief Description of the Drawings

The several features of the present invention will become more readily apparent from the fully detailed description taken in conjunction with the accompanying drawings in which:
Fig. 1 is a perspective view of a transducer of the invention;
Fig. 2 is a front view of the transducer shown in Fig. 1;
Fig. 3 is a front view of the reusable component of Fig. 1;
Fig. 4 is a cross-sectional view taken along lines 4-4 of Fig. 1;
Fig. 5 is an exploded, cross-sectional view taken along lines 5-5 of Fig. 2;
Fig. 6 is a rear, partially cut-away view of the disposable component of Fig. 1;
Figs. 7A-7C are diagrammatic side views, taken along lines 7A-7A of Fig. 2, to illustrate interaction of the dome wings and reusable component channels;
Fig. 8 is an exploded diagrammatic view of a pole mount arrangement for a plurality of the transducers of Fig. 1; and
Fig. 9 is a front diagrammatic view of a plurality of the transducers of Fig. 1 in pole-mounted position.

### Detailed Description of the Drawings

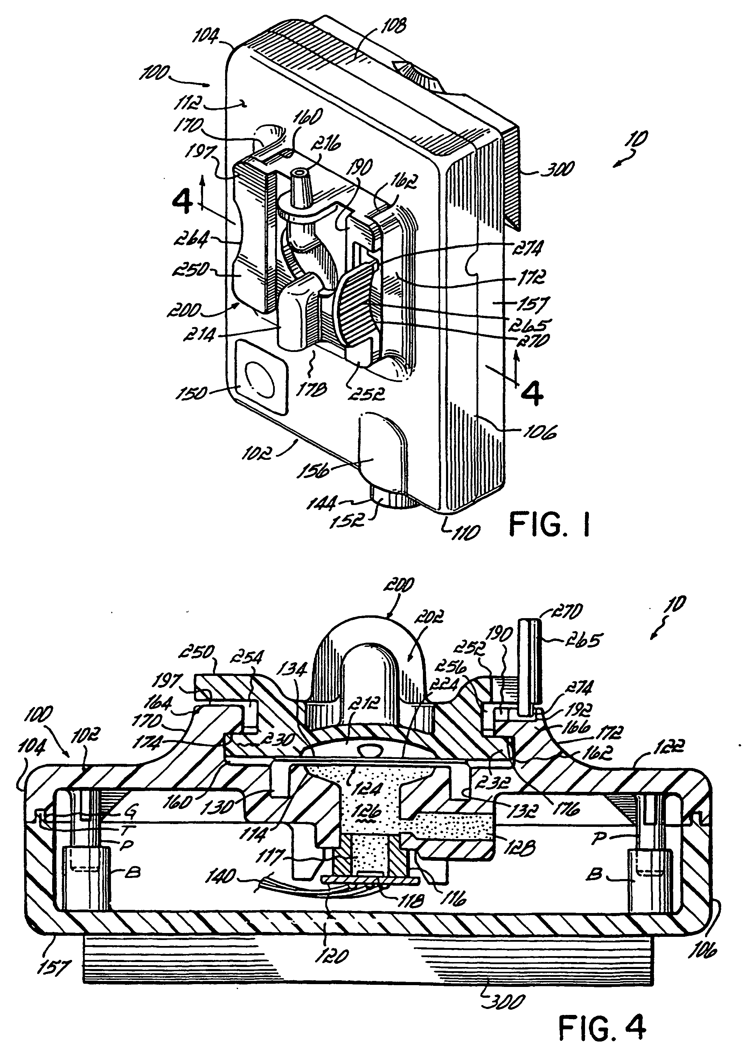

With reference to Fig. 1, there is shown a perspective view of a medical pressure transducer 10 of the present invention. Transducer 10 includes two major components, one being a reusable sensor component 100 of the invention and the other being a disposable fluid dome component 200 of the invention, removably and slidably mounted to reusable component 100.

With further reference to Figs. 1-5, reusable component 100 may be seen as having an opaque plastic support 102 in the form of a plate. Plate 102 has generally planar left edge 104, generally planar right edge 106, and generally planar top and bottom edges 108,110 to define a generally rectangular shape to plate 102. Extending between edges 104,106,108,110 is a generally planar front face 112.

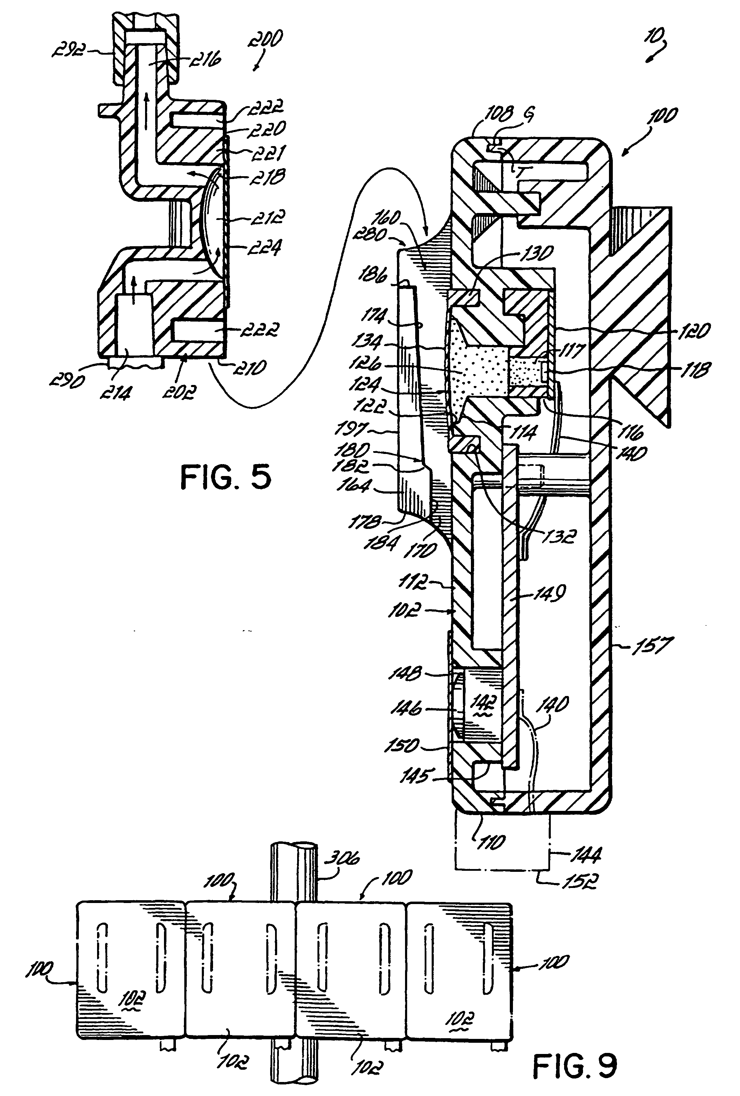

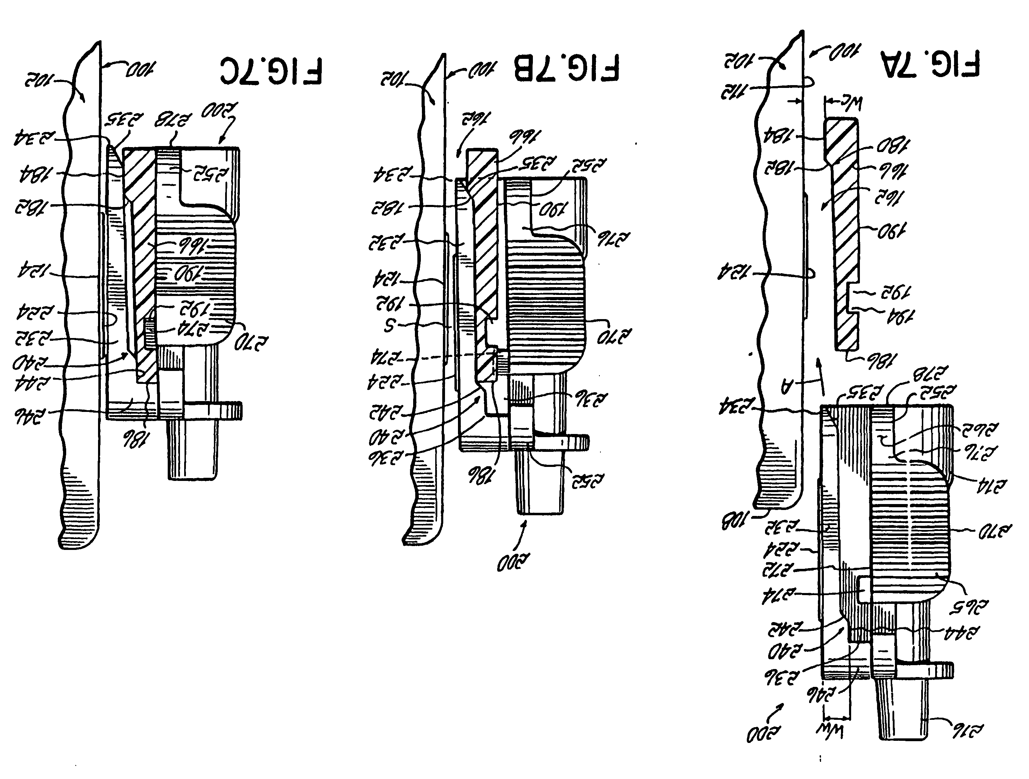

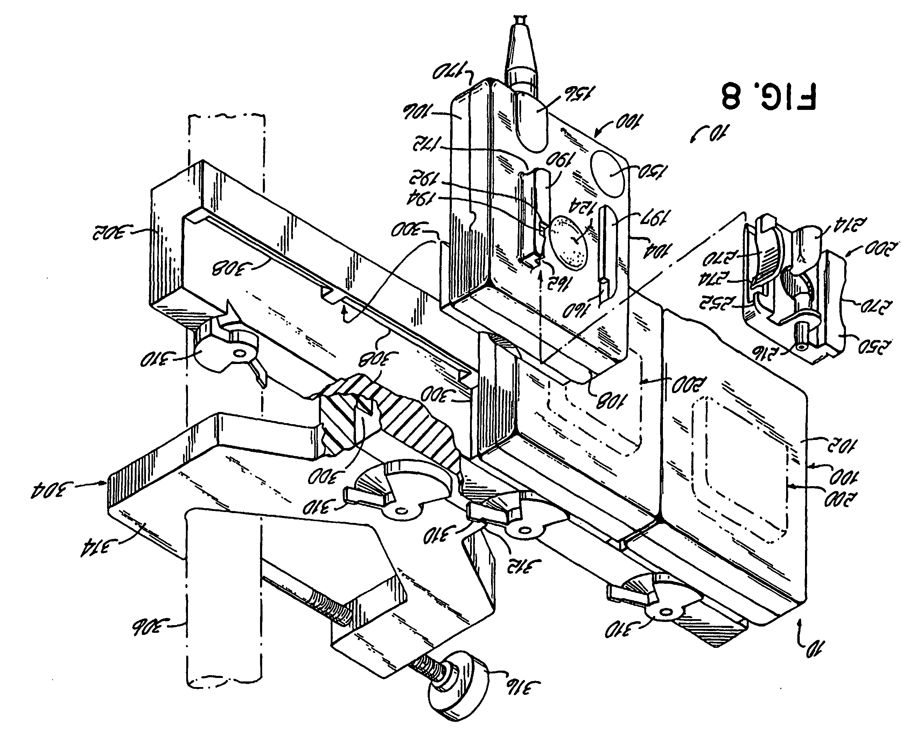

Extending from behind face 112 is an integral sensor chamber 114 (Figs. 4 and 5). Permanently attached within sensor chamber 114, such as by adhesive (not shown), is a plastic chimney 116 filled with cured gel 117 at the bottom of which is an integrated circuit sensor chip 118 mounted to printed circuit (PC) board substrate 120. PC board 120 contains appropriate circuitry thereon (not shown) and is affixed with chimney 116 to thus permanently associate sensor 118 with reusable component 100. At the upper end of chamber 114 through face 112 is an aperture 122. Permanently mounted over aperture 122 is an elastomeric reusable diaphragm 124 such as of molded polyurethane. Additional gel 126 is inserted in liquid state into chamber 114 between diaphragm 124 and chimney 116 via fill port 128 to bring diaphragm 124 into pressure communication, via gels 126 and 117, with sensor 118. Port 128 is sealed to thus slightly distend diaphragm 124 and gel 126 is cured.

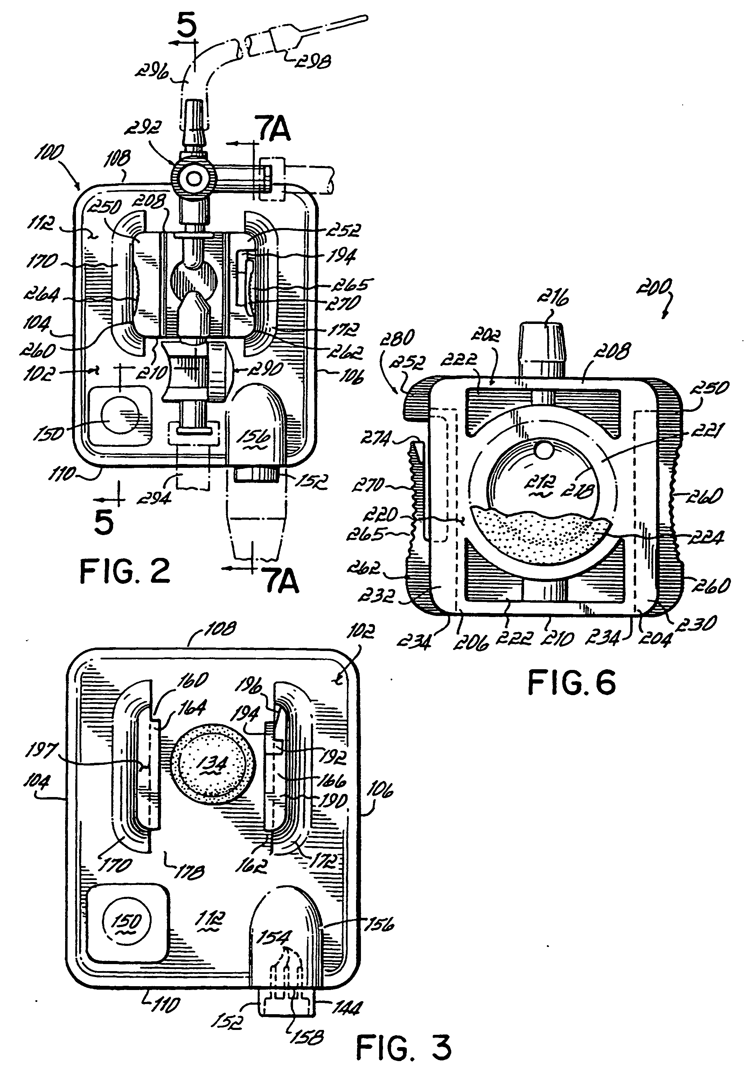

The edge 130 of diaphragm 124 defines a cylindrical collar that is fitted into annular groove 132 in face 112 about aperture 122 to hold diaphragm 124 to support 102 with the front face or surface 134 of reusable diaphragm 124 exposed in, or bulging just slightly above, the plane of front face 112. A plurality of conductors 140 interconnect calibration test switch 142 and connector 144 to PC board substrate 120 circuitry and sensor 118, all behind face 112 of plate 102. Switch 142 is fitted within open-bottom well 145 formed into face 112 with switch button 146 being accessible at aperture 148 through plate face 112 in the lower left corner thereof as seen from the front (Fig. 3). Conductors 140 could be separate wires or ribbon cable and/or conductive traces (not shown) on a switch-supporting PC board 149. Placed over aperture 148 is a compliant membrane 150 to protect switch 142 and to allow actuation thereof such as by gripping of reusable component 100 between the thumb and forefinger (not shown) in the area of membrane 150 and compressing same. Membrane 150 is adhesively held along its perimeter to the edge of well 145 defined at aperture 148. Actuation of switch 142 provides a calibration test as generally described in U.S. Patent 4760730, but as a directly integral part of the reusable component, rather than as a separate component.

To electrically connect to a monitor (not shown), connector 144 is provided at the bottom right of component 100 as seen from the front (Figs. 2 and 3). Connector 144 may have a cylindrical plastic shell 152 with female pin-receiving connectors 154 therein and housed in a bulbously protruded area 156 of component 100. Connector 144 is accessible through connector port 158 in bottom edge 110. Connector 144 may form part of a two-connector set as shown in U.S. Patent 5167522. An opaque plastic back plate 157 may be secured, such as by adhesive (not shown), over the back side of plate 102 to enclose the above-mentioned components.

To mount disposable dome component 200 as will be described, plate 102 is provided with a pair of channels 160,162 disposed to opposite left and right sides of reusable diaphragm 124 as seen from the front (Fig. 3). Each channel 160,162 is defined behind a respective outer front wall 164,166 associated with plate 102. To this end, outer front walls 164,166 are generally parallel to, but spaced from, front face 112 and held thereto by interconnecting side walls 170,172, respectively, to thus define channels 160,162 between front face 112 and the underside 174,176 of each respective outer front wall 164,166. The lateral extent of each channel 160,162 is further defined by side walls 170,172, respectively.

The underside 174 or 176 of outer front wall 164 or 166 may be slightly angled with a draft (such as for molding) as it progresses from near the top edge 108 of plate 102 towards the bottom edge 110 thereof (Fig. 5). The draft narrows somewhat the width of the channel 160 or 162 in the direction of insertion travel of the dome 200. For purposes described hereinafter, camming structure is provided at the terminal or bottom end 178 of the channels 160,162. The camming structure in the preferred embodiment shown is provided by camming ramp 180 comprised of a 45° ramp 182 and a trailing step 184 to define a generally precise channel width W_{c} thereat (see Fig. 7A). The top edge 186 of each wall 164,166 is exposed. The bottom end 178 of each channel 160 or 162 may be closed off (not shown) but is advantageously left open as shown so that debris does not accumulate in the channels.

With particular reference to Figs. 3 and 4, it may be seen that the outer surface 190 of right side channel outer front wall 166 includes a depression such as tab-receiving slot 192 formed therein (over side wall 172). Slot 192 extends into alleyway 194 also formed in outer surface 190 (over channel 162) which in turn ends adjacent chamfer 196 of outer front wall 166 all for purposes to be described hereinafter. Outer surface 190 is otherwise generally planar and parallel to front face 112. Outer surface 197 of left side, outer front wall 164 is similarly planar and parallel to front face 112.

With particular further reference to Figs. 5 and 6, disposable dome 200 is of clear or translucent plastic and may be seen as having a central body portion 202 defined between left and right edges 204,206 and top and bottom edges 208,210 to define a generally rectangular shape to central body portion 202. Formed centrally through the back of body portion 202 is a fluid path well 212 which communicates through an inlet port 214 extending up out of the front of well 212 and accessible along bottom edge 210 and outlet pipe 216 extending up out of the front of well 212 and beyond top edge 208. Inlet and outlet 214 and 216 cooperate to extend fluid path 212 through disposable dome 200. Fluid path 212 is accessible through a large aperture 218 along the back side 220 of central portion 202. Well 212 and aperture 218 are defined by a cylindrical wall 221 in central portion 202 with cavities 222 defined between wall 221 and edges 204,206,208,210. Alternatively, cavities 222 could be filled with plastic. Either way, back side 220 of dome 200 functions to define a flat or plate-like surface to dome 200 to match up to planar face 112 of plate 102.

Extending across aperture 218 along bottom side 220 is an elastomeric diaphragm 224 permanently affixed to central portion 202 and providing a pressure transmitting, fluid impervious wall to seal the fluid path within dome 200. Diaphragm 224 could be a molded polyurethane, like diaphragm 124 with a collar (not shown) mounted within an annular recess or groove (also not shown) about aperture 218. Alternatively, diaphragm 224 could be a sheet of urethane film material, the peripheral edge of which is either adhesively or thermally bonded to the edge of aperture 218, or is held into a groove (not shown) about aperture 218 such as by a compression ring (also not shown).

Extending outwardly from opposite left and right sides (as viewed in Figs. 2 and 4) of central portion 202 (from edges 204 and 206, respectively) and to either side of diaphragm 224 are left and right mounting wings 230,232 situated to be matingly received within channels 160,162 of reusable plate 102 such as to place disposable diaphragm 224 into confronting relationship with reusable diaphragm 124. Bottom end 234 of each wing 230,232 is chamfered as at 235 (see Fig. 7A) for purposes to be described hereinafter. Bottom end 234 just above chamfer 235 has a generally precise thickness or width, which in combination with diaphragms 224 and 124, is closely equal to channel width W_{c} so as to hold the diaphragms in proper pressure communicating relationship. Further camming structure is defined at top or distal end 236 of each wing. In the preferred embodiment shown, the further camming structure is provided by camming ramp 240 which, like the camming ramp 180 within channels 160 and 162, is comprised of a 45° ramp 242 and a trailing step 244 to define a precise width W_{w} of wing 230 or 232 in the area of trailing step 244. In this regard, the thickness or width of the channels 160,162 at their openings near the top edge 108 of plate 102 cooperate with diaphragms 224 and 124 to closely equal width W_{w} so as to, in addition or alternatively to the holding ability of bottom end 234 and width W_{c}, hold the diaphragms in proper pressure communicating relationship.

Camming ramp 180 and camming ramp 240 are situated on respective ones of the reusable component 100 and disposable dome 200 so as to engage their respective counterpart structures near the tailing end of the travel of dome 200 as the wings 230,232 are slidably received into the channels 160,162 of the reusable component 100. In this manner, as the dome 200 travels into reusable component 100, there may be a slight space (or just loose, sliding contact) between the diaphragms 124 and 224 so as not to harmfully abrade at least diaphragm 124. As dome 200 nears the end of its longitudinal travel into reusable component 100, wings 230,232 are driven, in a somewhat axial direction, towards face 112 and diaphragm 124 of plate 102 such that disposable diaphragm 224 is driven into abutting relationship with reusable diaphragm 124 to provide a proper pressure communicating relationship therebetween. To limit the extent of travel of dome 200 relative to plate 102, a closing wall 246 may be provided at distal or top end 236 of each wing 230,232 which closing wall 246 will abut into top edge 186 of outer front walls 164 or 166.

To facilitate use of dome 200 with reusable plate 102, a second set of wings 250,252 may be provided. Wings 250,252 also extend from edges 204 and 206 but spaced above respective ones of mounting wings 230,232 to define wall-receiving spaces 254,256 (Figs. 4 and 7). As is thus apparent, spaces 254,256 function as attachment structure channels of the dome to receive respective ones of outer walls 164 and 166 which function as wing-like attachment structure of the reusable component as dome 200 is slidingly received into reusable component 100. Second wings 250,252 extend generally outwardly so as to substantially overlie outer walls 164 and 166 to provide the aesthetic appearance of a single unit when components 200 and 100 are mounted together as shown in Fig. 1. Additionally, wings 250,252 may also provide a barrier to debris from entering channels 160,162 when dome 200 is mounted to plate 102.

As seen in Figs. 2 and 6, lateral edges 260,262 of second wings 250,252, respectively, may be indented as at 264 and 265, respectively, to provide finger and thumb gripping areas for the user (not shown) to grip dome 200 to mount and dismount same from reusable component 100. Finger-gripping portions 264, 265 may be textured (such as by grooving, serrating or knurling) to facilitate such manipulation by the user. With particular reference to Fig. 2, it may be seen that indented finger-gripping portion 265 of right side second wing 252 may actually be provided by resilient locking or flexing paddle 270 having an indented shape and a textured surface to simulate indented portion 264 of left side second wing 250. Supported at a terminal end 272 of paddle 270 is a locking tab 274 such that tab 274 is resiliently attached to dome 200 and spaced above and depending towards wing 232. Locking paddle 270 extends from a hinging area 276 nearer to the bottom end 278 of wing 252 such that by flexing action of locking paddle 270 locking tab 274 is movable towards central portion 202 near to the top end 280 of wine 252.

As dome 200 is slidably received into reusable component 100, locking tab 274 may bear against chamfer 196 (Fig. 3) of outer wall 166 to thereby flex paddle 270 leftwardly. As dome 200 moves further in its travel, tab 274 passes onto alleyway 194 and then, at the end of the travel of dome 200, snaps rightwardly back out into slot 192 (with a clicking sound) to lock dome 200 into position on plate 102 with diaphragms 224 and 124 in confronting, pressure transmitting relationship (Fig. 7C). To remove dome 200, the user (not shown) may grip dome 200 with the thumb (not shown) in indented portion 264 and the forefinger (not shown) against locking paddle 270 compressing same so that locking tab 274 comes away from tab-receiving slot 192, and then sliding upwardly towards the top edge 108 of plate 102 to withdraw dome 200 therefrom.

Dome 200 may be provided with a fast-flush device 290 coupled to inlet port 214 (such as the fast flush device shown in U.S. Patent 5171230) and a stopcock 292 coupled to outlet pipe 216. Flush device 290 may then be connected by tubing 294 to a source of saline (not shown) and stopcock 292 may be connected by further tubing 296 to a catheter 298 (Fig. 2) to be placed within the patient's circulatory system (not shown) to thus monitor the pressure thereof.

With reference to Figs. 7A-7C (in which channel 160 and its associated walls are removed for sake of clarity), there is shown diagrammatically the mounting of dome 200 to reusable component 100. In Fig. 7A, dome 200 is just about to be mounted to plate 102 with wing 232 just beginning to enter channel 162 in a direction along the downwardly-directed arrow A. Thus, dome wing 232 is coming into channel 162 from the direction of top edge 108 of plate 102. Top end 186 of outer front wall 166 may be impacted by chamfered wall 235 at the proximal end of wing 232 to help force wing 232 into the space or channel 162 defined behind outer front wall 166. In Fig. 7A, second wing 252 is spaced above and away from top surface 190 of front wall 166.

As dome 200 is continued in its downward progression towards bottom wall 110, as in Fig. 7B, most of the length of wing 232 passes into channel 162 and wing 252 passes over front 190 of front wall 166. In this progression of travel, it may be seen that there may be a slight space or at least a loose or sliding contact (indicated by the letter S) between diaphragms 224 and 124 so as to avoid damaging or chafing the diaphragms, and especially diaphragm 124 which is intended to be reusable with several of domes 200. Near the end of the travel, chamfer 235 hits against ramp 182 of camming ramp 180 to start to drive the proximal end of wing 232 towards face 112 and diaphragm 124. At about the same time, camming ramp 242 impacts against top edge 186 to also drive the distal end of wing 232 towards face 112 and diaphragm 124 in which event the spacing S between diaphragms 224 and 124 begins to decrease (or the loose contact begins to tighten up). Also, tab 274 impinges wall chamfer 196 (Fig. 3) and flexes paddle 270 inwardly so as to allow tab 274 to travel into alleyway 194.

In the end of the travel of dome 200 into reusable component 100 in Fig. 7C, the proximal end of wing 232 is situated below and against trailing end 184 and the top end 186 of outer wall 166 is situated above and against trailing end 244 of wing camming ramp 240 such that wing 232 has been driven towards plate face 112 and diaphragms 224 and 124 have been driven into abutting relationship to provide the desired pressure communicating relationship therebetween. Also, in this terminal end of the travel, second wing 252 is positioned so as to substantially completely overlie top surface 190 of front wall 166, and paddle 270 has gone back towards its original position with tab 274 locked into slot 192. The same arrangement of travel as shown in Figs. 7A-7C occurs simultaneously between wing 230 and channel 160.

In use, dome 200 is slidably mounted to reusable component 100 as above described and appropriate tubing 294,296 and catheter 298 are employed to couple fluid path 212 of transducer 10 to a patient and connector 144 utilized to couple signals representing the patient's blood pressure, for example, with a monitor in an otherwise conventional manner. After the use for that patient is completed, or should dome 200 need to be replaced for any reason, dome 200 may be simply removed by depressing locking paddle 270 and sliding dome 200 out of channels 160,162 of reusable component 100 and the dome 200 disposed of (with or without tubing). Either new tubing may be provided, or the old tubing used, with a new dome 200 as appropriate, depending upon the patient's situation, and new dome 200 slidably remounted to reusable component 100 as previously described. In many situations, it may be desirable to monitor more than one pressure. In this event, multiple transducers 10 may be utilized as will now be described with reference to Fig. 8.

Back plate 157 of each reusable component may be provided with a mounting structure 300 to mount component 100 to a support frame 302 which, in turn, is mounted to a pole-mount clamp 304 secured to a pole 306. The mounting frame 302 includes a plurality of receptacles 308 to receive the respective support structure 300 of a plurality of reusable components 100 which are then locked in place by actuation of the locking handle 310 on frame 302. Frame 302 also includes an identical mounting structure 300 receivable in an identical receptacle 308 and held thereto by actuation of handle 310 on the proximal end 312 of pole mount clamp 304. Clamp 304 is held to pole 306 in conventional manner such as by interaction of yoke 314 and screw 316 about pole 306. Alternatively, or additionally, each plate 102 could be provided as modular interconnecting plates as shown in International Patent Application WO95/01195.

Due to the rectangular nature of plate 102, it may be seen that when a plurality of reusable components are mounted as above-described (see Fig. 9), they give the appearance of being a solid set of units, more or less, and may thus be considered as being modular in that any one of the reusable components 100 may be placed in any one of the positions defined by receptacles 308 available on frame 302. Domes 200 may be mounted to their respective reusable components 100 either before or after the related component 100 is connected to frame 302. Alternatively, one reusable component 100 may be mounted directly to the pole mount clamp 304 and frame 302 dispensed with.

By virtue of the foregoing, there is thus provided a medical pressure transducer with a disposable fluid dome, slidably removable to a reusable sensor component with an enhanced pressure transmitting relationship between the diaphragms and with improvements to facilitate operator use of the transducer. While the use of two wings and two channels is shown in the preferred embodiment, at least one of each may be employed. Also, the outer front walls may be coplanar with front face 112 with appropriate adjustment in the elevation of either diaphragm 124 or mounting wings 230,232, by way of example. Further, while slot 192 is shown on support 102 and locking tab 274 on dome 200, they could be reversed.

## Claims

1. A medical pressure transducer comprising a reusable component (100) for use with a disposable dome (200) having a fluid path (212) adapted to be coupled to a patient, a diaphragm (224) coupled to said fluid path, and at least one mounting wing (230, 232) extending to one side of said dome diaphragm, the reusable component comprising a support (102), a pressure sensor (118) permanently associated with the support, a reusable diaphragm (124) in pressure communication with the sensor and at least one channel (160, 162) disposed to one side of the reusable diaphragm for slidably receiving said dome wing such that said dome diaphragm is slid by the reusable diaphragm and slidably received into confronting relationship with the reusable diaphragm, **characterised by** one of a depression (192) and a projection (274) associated with the support and being relatively positioned to receive a projection (274) or be received in a depression (192), respectively, associated with said dome (200) as said dome wing (230, 232) is slidably received into the channel (160, 162) to lock said disposable diaphragm into confronting relationship with the reusable diaphragm.

2. A medical pressure transducer comprising a disposable dome (200) for use with a reusable component (100) having a support (102), a pressure sensor (118) permanently associated with said support, a reusable diaphragm (124) in pressure communication with said sensor and at least one channel (160, 162) disposed to one side of said reusable diaphragm, the disposable dome comprising a fluid path (212) adapted to be coupled to a patient, a diaphragm (224) coupled to the fluid path, and at least one mounting wing (230, 232) extending to one side of the dome diaphragm and slidably receivable in said reusable component channel such that the dome diaphragm slid by the reusable diaphragm and is slidably received into confronting relationship with said reusable diaphragm, **characterised by** one of a projection (274) and a depression (192) associated with the dome (200) and being relatively positioned to be received in a depression (192) or receive a projection (274), respectively, associated with said support as the wing is slidably received into said channel to lock the disposable diaphragm into confronting relationship with said reusable diaphragm.

3. The transducer as claimed in Claim 2 further comprising a reusable component (100) having a support (102), a pressure sensor (118) permanently associated with the support, a reusable diaphragm (124) in pressure communication with the sensor and at least one channel (160, 162) disposed to one side of the reusable diaphragm for slidably receiving the dome wing such that the dome diaphragm is slidably received into confronting relationship with the reusable diaphragm; **characterised by** both a depression (192) and a projection (274) each associated with a respective one of the support and the dome (200), the depression (192) and projection (274) being relatively positioned such that the depression receives the projection as the wing (230, 232) is slidably received into the channel (160, 162) to lock the disposable diaphragm into confronting relationship with the reusable diaphragm.

4. The transducer as claimed in either Claim 2 or Claim 3, wherein the projection (274) is associated with the disposable dome (200).

5. The transducer as claimed in any of Claims 2 to 4 wherein the depression is a tab-receiving slot (192) associated with the support (102) and the projection is a locking tab (274) associated with the dome (200).

6. The transducer as claimed in any preceding Claim further **characterized by** a flexing paddle (270) associated with the dome, the projection (274) being supported by the flexing paddle (270).

7. The transducer as claimed in Claim 6 wherein the projection (274) is supported at a terminal end (272) of the flexing paddle (270).

8. The transducer as claimed in either Claim 1 or Claim 3 wherein the channel (160, 162) is defined behind an outer front wall (164, 166).

9. The transducer as claimed in Claim 8 wherein the depression (192) is defined in the outer front wall (164, 166).

10. The transducer as claimed in either Claim 8 or Claim 9 further **characterised by** a second wing (250, 252) spaced above the mounting wing (230, 232) to define a wall-receiving space (254, 256) for the outer front wall (164, 166).

11. The transducer as claimed in Claim 10 further **characterized by** a projection (274) associated with the second wing (250, 252) and receivable in a depression (192) defined in the reusable component (100).

12. The transducer as claimed in either Claim 10 or 11 further **characterized by** a finger gripping portion (264, 265) defined in the second wing (250, 252).

13. The transducer as claimed in any of Claims 10 to 12 wherein the finger gripping portion (264, 265) is defined along a lateral edge (260, 262) of the second wing (250, 252).

14. The transducer as claimed in any of Claims 10 to 13 wherein the second wing (250, 252) extends parallel to the mounting wing (230, 232).

15. The transducer as claimed in any of Claims 10 to 14 wherein the second wing (250, 252) defines an edge (260, 262) by which to facilitate removal of the disposable dome (200) from the reusable component (100).

16. The transducer as claimed in Claim 3 or any one of Claims 4 to 15 as dependent on Claim 3 wherein the reusable component (100) includes two such channels (160, 162) and the disposable dome (200) includes two such mounting wings (230, 232).

17. The transducer as claimed in Claim 16 wherein the channels (260, 262) are disposed to opposite sides of the reusable diaphragm (124) and the wings (230, 232) are disposed to opposite sides of the disposable diaphragm (224).

18. The transducer as claimed in Claim 1 or Claim 3 or any one of Claims 4 to 17 as dependent on either Claim 1 or Claim 3 further comprising a calibration test switch (142) associated with the support (102) and electrically connected to the sensor (118).

19. The transducer as claimed in Claim 3 or any one of Claims 4 to 18 as dependent on Claim 3 wherein the support (102) includes peripheral side edges (104, 106, 108, 110) that define a generally rectangular shape and a front face (112) whereat the reusable diaphragm (124) is exposed.

## Patentansprüche

1. Medizinischer Druckwandler mit einer wieder verwendbaren Komponente (100) zur Verwendung mit einem Einwegaufsatz (200), der eine an einen Patienten anschließbare Fluidbahn (212), eine mit der Fluidbahn verbundene Membran (224) und wenigstens eine sich nach einer Seite der Aufsatzmembran erstreckende Montageflanke (230, 232) aufweist, wobei die wieder verwendbare Komponente aufweist einen Sockel (102), einen dauerhaft mit dem Sockel verbundenen Drucksensor (118), eine wieder verwendbare Membran (124) in Druckverbindung mit dem Sensor und wenigstens eine Nut (160, 162), die an einer Seite der wieder verwendbaren Membran angeordnet ist, um die Aufsatzflanke gleitend aufzunehmen, derart, dass die Aufsatzmembran an der wieder verwendbaren Membran vorbeigleitet und gleitend eine zu der verwendbaren Membran gegenüberliegende Position erreicht, **gekennzeichnet durch** eine Vertiefung (192) oder einen Vorsprung (274), die dem Sockel zugeordnet und so zueinander positioniert sind, dass sie einen Vorsprung (274) aufnehmen bzw. in einer Vertiefung (192) aufgenommen werden, die dem Aufsatz (200) zugeordnet sind, wenn die Aufsatzflanke (230, 232) gleitend in der Nut (160, 162) aufgenommen ist, um die Einwegmembran in einer gegenüber liegenden Position mit der wieder verwendbaren Membran zu verriegeln.

2. Medizinischer Druckwandler mit einem Einwegaufsatz (200) zur Verwendung mit einer wieder verwendbaren Komponente (100), die einen Sockel (102), einen dauerhaft mit dem Sockel verbundenen Drucksensor (118), eine wieder verwendbaren Membran (124) in Druckverbindung mit dem Sensor, und wenigstens eine Nut (160, 162), die an einer Seite der wieder verwendbaren Membran angeordnet ist, wobei der Einwegaufsatz aufweist eine Fluidbahn (212), die den Anschluss an einen Patienten ermöglicht, eine Membran (224), die mit der Fluidbahn verbunden ist, und wenigstens eine Montageflanke (230, 230), die sich nach einer Seite der Aufsatzmembran erstreckt und gleitend in der Nut der wieder verwendbaren Komponente aufnehmbar ist, derart, dass die Aufsatzmembran an der wieder verwendbaren Membran vorbeigleitet und gleitend eine gegenüberliegende Beziehung mit der wieder verwendbaren Membran erreicht, **gekennzeichnet durch** einen Vorsprung (274) oder eine Vertiefung (192), die dem Aufsatz (200) zugeordnet und so zueinander positioniert sind, dass sie in einer Vertiefung (192) aufgenommen werden bzw. einen Vorsprung (274) aufnehmen, der dem Sockel zugeordnet ist, wenn die Flanke gleitend in der Nut aufgenommen ist, um die Einwegmembran in einer gegenüberliegenden Beziehung mit der wieder verwendbaren Membran zu verriegeln.

3. Wandler nach Anspruch 2, weiterhin mit einer wieder verwendbaren Komponente (100), die einen Sockel (102) aufweist, einem dauerhaft mit dem Sockel verbundenen Drucksensor (118), einer wieder verwendbaren Membran (124) in Druckverbindung mit dem Sensor und wenigstens einer Nut (160, 162), die an einer Seite der wieder verwendbaren Membran zum gleitenden Aufnehmen der Aufsatzflanke angeordnet ist, derart, dass die Aufsatzmembran gleitend in einer gegenüberliegenden Beziehung zu der wieder verwendbaren Membran aufgenommen wird, **gekennzeichnet durch** sowohl eine Vertiefung (192) als auch einen Vorsprung (274), die jeweils sowohl dem Sockel als auch dem Aufsatz (200) zugeordnet sind, wobei die Vertiefung (192) und der Vorsprung (274) so zueinander positioniert sind, dass die Vertiefung den Vorsprung aufnimmt, wenn die Flanke (230, 232) gleitend in der Nut (160, 162) aufgenommen ist, um die Einwegmembran mit der wieder verwendbaren Membran in einer gegenoberliegenden Beziehung zu verriegeln.

4. Wandler nach Anspruch 2 oder 3, bei dem der Vorsprung (274) mit dem Einwegaufsatz (200) verbunden ist.

5. Wandler nach einem der Ansprüche 2 bis 4, bei dem die Vertiefung ein Nasenaufnehmender Schlitz (192) ist, der mit dem Sockel (102) verbunden ist, und der Vorsprung eine mit dem Aufsatz (200) verbundene Verriegelungsnase (274) ist.

6. Wandler nach einem der vorangegangenen Ansprüche, **gekennzeichnet durch** einen mit dem Aufsatz verbundenen flexiblen Tasthebel (270), wobei der Vorsprung (274) von dem flexiblen Tasthebel (270) gehalten wird.

7. Wandler nach Anspruch 6, bei dem der Vorsprung (274) an einer Endseite (272) des flexiblen Tasthebels (270) gehalten wird.

8. Wandler nach Anspruch 1 oder 3, bei dem die Nut (160, 162) hinter einer äußeren Vorderwand (164, 166) ausgebildet ist.

9. Wandler nach Anspruch 8, bei dem die Vertiefung (192) in der äußeren Vorderwand (164, 166) ausgebildet ist.

10. Wandler nach Anspruch 8 oder 9, weiterhin **gekennzeichnet durch** eine zweite Flanke (250, 252), die in einem Abstand über der Montageflanke (230, 232) angeordnet ist, um einen Wandaufnahme-Raum (264, 256) für die äußere Vorderwand (164, 166) zu bilden.

11. Wandler nach Anspruch 10, weiterhin **gekennzeichnet durch** einen Vorsprung (274), der mit der zweiten Flanke (250, 252) verbunden ist und in einer Vertiefung (192) aufnehmbar ist, die in der wieder verwendbaren Komponente (100) ausgebildet ist.

12. Wandler nach Anspruch 10 oder 11, weiterhin **gekennzeichnet durch** einen Griffbereich (264, 265), der in der zweiten Flanke (250, 252) ausgebildet ist.

13. Wandler nach einem der Ansprüche 10 bis 12, bei dem der Griffbereich (264, 265) entlang einer Seitenkante (260, 262) des zweiten Flügels (250, 252) ausgebildet ist.

14. Wandler nach einem der Ansprüche 10 bis 13, bei dem sich die zweite Flanke (250, 252) parallel zur Montageflanke (230, 232) erstreckt.

15. Wandler nach einem der Ansprüche 10 bis 14, bei dem die zweite Flanke (250, 252) eine Kante (260, 262) bildet, durch welche die Abnahme des Einwegaufsatzes (200) von der wieder verwendbaren Komponente (100) erleichtert wird.

16. Wandler nach Anspruch 3 oder einem der Ansprüche 4 bis 15, sofern abhängig von Anspruch 3, bei dem die wieder verwendbare Komponente (100) zwei solcher Nuten (160, 162) und der Einwegaufsatz (200) zwei solcher Montageflanken (230, 232) umfasst.

17. Wandler nach Anspruch 16, bei dem die Nuten (260, 262) an den gegenüberliegenden Seiten des wieder verwendbaren Aufsatzes (124) angebracht und die Flanken (230, 232) an den gegenüberliegenden Seiten der Einwegmembran (224) angebracht sind.

18. Wandler nach Anspruch 1 oder 3 oder einem der Ansprüche 4 bis 17, sofern abhängig entweder von Anspruch 1 oder 3, weiterhin mit einem Kalibrationstest-Schalter (142), der dem Sockel (102) zugeordnet und mit dem Sensor (118) elektrisch verbunden ist.

19. Wandler nach Anspruch 3 oder einem der Ansprüche 4 bis 18, sofern abhängig von Anspruch 3, bei dem der Sockel (102) aufweist periphere Seitenkanten (104, 106, 108, 110), die eine im Wesentlichen rechteckige Form bilden, und eine Vorderwand (112), an welcher die wieder verwendbare Membran (124) freiliegt.

## Revendications

1. Transducteur de pression médical comportant un composant réutilisable (100) destiné à être utilisé avec un dôme jetable (200) ayant un trajet de fluide (212) adapté pour être couplé à un patient, un diaphragme (224) couplé audit trajet de fluide, et au moins une ailette de montage (230, 232) s'étendant d'un côté dudit diaphragme de dôme, le composant réutilisable comportant un support (102), un capteur de pression (118) associé de manière permanente au support, un diaphragme réutilisable (124) en communication de pression avec le capteur et au moins un canal (160, 162) disposé d'un côté du diaphragme réutilisable pour recevoir ladite ailette de dôme de manière coulissante, de telle sorte que ledit diaphragme de dôme est coulissé par l'intermédiaire du diaphragme réutilisable, et est reçu de manière coulissante dans une relation en vis-à-vis avec le diaphragme réutilisable, **caractérisé par** un élément parmi un enfoncement (192) et une saillie (274) associé au support, et étant positionné de manière relative pour recevoir une saillie (274) ou être reçu dans un enfoncement (192), respectivement, associé(e) audit dôme (200) lorsque ladite ailette de dôme (230, 232) est reçue de manière coulissante dans le canal (160, 162) pour verrouiller ledit diaphragme jetable dans une relation en vis-à-vis avec le diaphragme réutilisable.

2. Transducteur de pression médical comportant un dôme jetable (200) destiné à être utilisé avec un composant réutilisable (100) ayant un support (102), un capteur de pression (118) associé de manière permanente audit support, un diaphragme réutilisable (124) en communication de pression avec ledit capteur et au moins un canal (160, 162) disposé d'un côté dudit diaphragme réutilisable, le dôme jetable comportant un trajet de fluide (212) adapté pour être couplé à un patient, un diaphragme (224) couplé au trajet de fluide, et au moins une ailette de montage (230, 232) s'étendant d'un côté du diaphragme de dôme et pouvant être reçue de manière coulissante dans ledit canal de composant réutilisable, de telle sorte que le diaphragme de dôme coulisse par l'intermédiaire du diaphragme réutilisable, et est reçu de manière coulissante dans une relation en vis-à-vis avec ledit diaphragme réutilisable, **caractérisé par** un élément parmi une saillie (274) et un enfoncement (192) associé au dôme (200), et étant positionné de manière relative pour être reçu dans un enfoncement (192) ou pour recevoir une saillie (274), respectivement, associé(e) audit support lorsque l'ailette est reçue de manière coulissante dans ledit canal pour verrouiller le diaphragme jetable dans une relation en vis-à-vis avec ledit diaphragme réutilisable.

3. Transducteur selon la revendication 2, comportant en outre un composant réutilisable (100) ayant un support (102), un capteur de pression (118) associé de manière permanente au support, un diaphragme réutilisable (124) en communication de pression avec le capteur, et au moins un canal (160, 162) disposé d'un côté du diaphragme réutilisable pour recevoir l'ailette de dôme de manière coulissante, de telle sorte que le diaphragme de dôme est reçu de manière coulissante dans une relation en vis-à-vis avec le diaphragme réutilisable, **caractérisé par** un enfoncement (192) et une saillie (274) associés chacun à un élément respectif parmi le support et le dôme (200), l'enfoncement (192) et la saillie (274) étant positionnés de manière relative de telle sorte que l'enfoncement reçoit la saillie lorsque l'ailette (230, 232) est reçue de manière coulissante dans le canal (160, 162) pour verrouiller le diaphragme jetable dans une relation en vis-à-vis avec le diaphragme réutilisable.

4. Transducteur selon la revendication 2 ou 3, dans lequel la saillie (274) est associée au dôme jetable (200).

5. Transducteur selon l'une quelconque des revendications 2 à 4, dans lequel l'enfoncement est une fente de réception de patte (192) associée au support (102), et la saillie est une patte de verrouillage (274) associée au dôme (200).

6. Transducteur selon l'une quelconque des revendications précédentes, **caractérisé en outre par** une palette flexible (270) associée au dôme, la saillie (274) étant supportée par la palette flexible (270).

7. Transducteur selon la revendication 6, dans lequel la saillie (274) est supportée au niveau d'une extrémité terminale (272) de la palette flexible (270).

8. Transducteur selon la revendication 1 ou 3, dans lequel le canal (160, 162) est défini dernière une paroi avant extérieure (164, 166).

9. Transducteur selon la revendication 8, dans lequel l'enfoncement (192) est défini dans la paroi avant extérieure (164, 166).

10. Transducteur selon la revendication 8 ou 9, **caractérisé en outre par** une seconde ailette (250, 252) espacée au-dessus de l'ailette de montage (230, 232) pour définir un espace de réception de paroi (254, 256) destiné à la paroi avant extérieure (164, 166).

11. Transducteur selon la revendication 10, **caractérisé en outre par** une saillie (274) associée à la seconde ailette (250, 252), et pouvant être reçue dans un enfoncement (192) défini dans le composant réutilisable (100).

12. Transducteur selon la revendication 10 ou 11, **caractérisé en outre par** une partie de préhension manuelle (264, 265) définie dans la seconde ailette (250, 252).

13. Transducteur selon l'une quelconque des revendications 10 à 12, dans lequel la partie de préhension manuelle (264, 265) est définie le long d'un bord latéral (260, 262) de la seconde ailette (250, 252).

14. Transducteur selon l'une quelconque des revendications 10 à 13, dans lequel la seconde ailette (250, 252) s'étend parallèlement à l'ailette de montage (230, 232).

15. Transducteur selon l'une quelconque des revendications 10 à 14, dans lequel la seconde ailette (250, 252) définie un bord (260, 262) qui facilite un enlèvement du dôme jetable (200) à partir du composant réutilisable (100).

16. Transducteur selon la revendication 3 ou l'une quelconque des revendications 4 à 15 lorsque dépendante de la revendication 3, dans lequel le composant réutilisable (100) comporte deux canaux (160, 162), et le dôme jetable (200) comporte deux ailettes de montage (230, 232).

17. Transducteur selon la revendication 16, dans lequel les canaux (260, 262) sont disposés sur des côtés opposés du diaphragme réutilisable (124), et les ailettes (230, 232) sont disposées sur des côtés opposés du diaphragme jetable (224).

18. Transducteur selon la revendication 1 ou 3 ou l'une quelconque des revendications 4 à 17 lorsque dépendante de la revendication 1 ou de la revendication 3, comportant en outre un commutateur de test de calibrage (142) associé au support (102), et connecté électriquement au capteur (118).

19. Transducteur selon la revendication 3 ou l'une quelconque des revendications 4 à 18 lorsque dépendante de la revendication 3, dans lequel le support (102) comporte des bords latéraux périphériques (104, 106, 108, 110) qui définissent une forme généralement rectangulaire, et une face avant (112) au niveau de laquelle le diaphragme réutilisable (124) est exposé.
